# EUROPÄISCHE PATENTANMELDUNG

(11) **EP 4 814 989 A1**
(43) Veröffentlichungstag der Anmeldung: **30.09.2026**
(21) Anmeldenummer: 25167049.3
(22) Anmeldetag: 28.03.2025
(51) Int. Cl.: G16H 70/20, G16H 50/70, G16H 50/20, G16H 40/20

(54) **COMPUTERIMPLEMENTIERTES VERFAHREN ZU EINEM BEREITSTELLEN EINER EMPFEHLUNGSINFORMATION FÜR ZUMINDEST EIN MAGNETRESONANZ-PROTOKOLL FÜR EINE MAGNETRESONANZUNTERSUCHUNG**

(71) Anmelder: Siemens Healthineers AG, 91301 Forchheim (DE)
(72) Erfinder: Wagner, Fabian, 91052 Erlangen (DE); Würfl, Tobias, 91054 Erlangen (DE); Wohlers, Julian, 91058 Erlangen (DE); Schneider, Rainer, 91315 Höchstadt (DE)
(74) Vertreter: Siemens Healthineers Patent Attorneys

(57) **Zusammenfassung**

Computerimplementiertes Verfahren zu einem Bereitstellen einer Empfehlungsinformation für zumindest ein Magnetresonanz-Protokoll für eine Magnetresonanzuntersuchung

Die Erfindung geht aus von einem Verfahren Computerimplementiertes Verfahren zu einem Bereitstellen einer Empfehlungsinformation für zumindest ein Magnetresonanz-Protokoll für eine Magnetresonanzuntersuchung an einem Patienten, umfassend die folgenden Verfahrensschritte:
- Bereitstellen von zumindest einer untersuchungsrelevanten Patienteninformation und/oder zumindest einer untersuchungsrelevanten Anwenderinformation,
- Bereitstellen von Protokollinformationen zu unterschiedlichen Magnetresonanz-Protokollen aus einer Gesamtheit an zur Verfügung stehenden Magnetresonanz-Protokollen,
- Anwenden eines trainierten Sprachen-Modells auf die zumindest eine untersuchungsrelevanten Patienteninformation und/oder die zumindest eine untersuchungsrelevanten Anwenderinformation zur Ermittlung eines Patienten-Merkmalsvektors,
- Anwenden des trainierten Sprachen-Modells auf die unterschiedlichen Magnetresonanz-Protokolle zur Ermittlung eines Protokoll-Merkmalsvektors für jedes der unterschiedlichen Magnetresonanz-Protokolle wird,
- Anwenden eines Ähnlichkeits-Algorithmus auf den Patienten-Merkmalsvektor und den unterschiedlichen Protokoll-Merkmalsvektoren und Ermitteln einer Empfehlungsinformation umfassend zumindest ein Magnetresonanz-Protokoll, und,
- Bereitstellen der Empfehlungsinformation.

## Beschreibung

Die vorliegende Erfindung betrifft ein computerimplementiertes Verfahren zu einem Bereitstellen einer Empfehlungsinformation für zumindest ein Magnetresonanz-Protokoll für eine Magnetresonanzuntersuchung an einem Patienten. Des Weiteren betrifft die Erfindung eine Recheneinheit zu einem Regeln und/oder Steuern eines Verfahrens zu einem Bereitstellen einer Empfehlungsinformation für zumindest ein Magnetresonanz-Protokoll für eine Magnetresonanzuntersuchung an einem Patienten. Die Erfindung betrifft weiterhin ein System zu einem Bereitstellen einer Empfehlungsinformation für zumindest ein Magnetresonanz-Protokoll für eine Magnetresonanzuntersuchung an einem Patienten. Ein weiterer Aspekt der Erfindung umfasst ein Computerprogrammprodukt mit Programmelementen zu einem Ausführen des Verfahrens zu einem Bereitstellen einer Empfehlungsinformation für zumindest ein Magnetresonanz-Protokoll für eine Magnetresonanzuntersuchung.

Unabhängig vom grammatikalischen Geschlecht eines bestimmten Begriffes sind Personen mit männlicher, weiblicher oder anderer Geschlechteridentität mit umfasst.

Zur Klärung von klinischen und/oder diagnostischen Fragestellungen an Patienten werden häufig Magnetresonanzuntersuchung am Patienten durchgeführt. Hierzu wird ein Magnetresonanz-Protokoll von einem medizinischen Bedienpersonal erstellt und/oder ausgewählt, wobei das Magnetresonanz-Protokoll einen Untersuchungsablauf der Magnetresonanzuntersuchung vorgibt. Ein derartiges Magnetresonanz-Protokoll umfasst bevorzugt mehrere Magnetresonanz-Sequenzen, die in einer definierten Reihenfolge nacheinander ausgeführt werden.

Bei der Auswahl eines Magnetresonanz-Protokolls sollen die zu klärende klinische und/oder diagnostische Fragestellung und/oder eine abzubildende Anatomie und/oder weitere Patientendaten von dem medizinischen Bedienpersonal berücksichtigt werden. Zudem besteht die Möglichkeit, bei der Auswahl eines Magnetresonanz-Protokolls eine bestimmte Scan-Strategie, beispielsweise eine besonders schnelle Messung oder eine Messung mit einer besonders hohen Bildauflösung oder auch eine besonders leise Messung, auszuwählen. Des Weiteren kann bei einer ausgewählten Scan-Strategie weiter differenziert werden, beispielsweise ob ein Kontrastmittel während der Messung dem Patienten verabreicht werden soll oder ob bei der Messung ein Implantat des Patienten berücksichtigt werden muss usw.

Bisher wird eine Planung einer Magnetresonanzuntersuchung und damit eine Auswahl eines Magnetresonanz-Protokolls für die Magnetresonanzuntersuchung von einem medizinischen Bedienpersonal, beispielsweise einem Radiologen oder einem Arzt, ausgeführt. Dies ist jedoch ein sehr zeitaufwendiger Prozess, der viel Erfahrung des medizinischen Bedienpersonals erfordert. Beispielsweise erfordert eine Auswahl eines Magnetresonanz-Protokolls eine genaue Identifizierung der Magnetresonanz-Protokolle aus einem Protokollbaum, der in einer Protokoll-Datenbank einer Magnetresonanzvorrichtung gespeichert und/oder konfiguriert ist. Eine Auswahl des Magnetresonanz-Protokolls wird meist von einem Assistenten durchgeführt, der wiederum ebenfalls eine genaue Kenntnis des Protokollbaums und einer Bezeichnung und/oder Funktionsweise der einzelnen Magnetresonanz-Protokolle des Protokollbaums besitzen sollte. Zudem ist hierbei auch eine Berücksichtigung von untersuchungsrelevanten Patienteninformationen sinnvoll, beispielsweise wenn eine Patientenbewegung erwartet wird. Dies kann jedoch zu Inkonsistenzen und nicht standardisierten Protokollauswahlen führen. Zudem kann bei einem unerfahrenen medizinischen Bedienpersonal eine falsche Protokollzuordnung erfolgen, die zu unbrauchbaren Messdaten und/oder zu einer zeitaufwendigen Wiederholung der Magnetresonanzuntersuchung führen kann.

Der vorliegenden Erfindung liegt insbesondere die Aufgabe zugrunde, eine zuverlässige und schnelle Unterstützung eines medizinischen Bedienpersonal bei der Auswahl eines Magnetresonanz-Protokolls bereitzustellen. Die Aufgabe wird durch die Merkmale der unabhängigen Ansprüche gelöst. Vorteilhafte Ausgestaltungen sind in den Unteransprüchen beschrieben.

Die Erfindung geht aus von einem computerimplementierten Verfahren zu einem Bereitstellen einer Empfehlungsinformation für zumindest ein Magnetresonanz-Protokoll für eine Magnetresonanzuntersuchung an einem Patienten, umfassend die folgenden Verfahrensschritte:
- Bereitstellen von zumindest einer untersuchungsrelevanten Patienteninformation und/oder zumindest einer untersuchungsrelevanten Anwenderinformation,
- Bereitstellen von Protokollinformationen zu unterschiedlichen Magnetresonanz-Protokollen aus einer Gesamtheit an zur Verfügung stehenden Magnetresonanz-Protokollen,
- Anwenden eines trainierten Sprachen-Modells auf die zumindest eine untersuchungsrelevanten Patienteninformation und/oder die zumindest eine untersuchungsrelevanten Anwenderinformation, wobei mittels des trainierten Sprachen-Modells ein Patienten-Merkmalsvektor ermittelt und bereitgestellt wird,
- Anwenden eines trainierten Sprachen-Modells auf die Protokollinformationen der unterschiedlichen Magnetresonanz-Protokolle, wobei mittels des trainierten Sprachen-Modells ein Protokoll-Merkmalsvektor für jedes Magnetresonanz-Protokoll der unterschiedlichen Magnetresonanz-Protokolle ermittelt und bereitgestellt wird,
- Anwenden eines Ähnlichkeits-Algorithmus auf den Patienten-Merkmalsvektor und den unterschiedlichen Protokoll-Merkmalsvektoren und Ermitteln einer Empfehlungsinformation umfassend zumindest ein Magnetresonanz-Protokoll, wobei der Protokoll-Merkmalsvektor des zumindest einen Magnetresonanz-Protokolls der Empfehlungsinformation eine Maximal-Übereinstimmung zu dem Patienten-Merkmalsvektor aufweist, und
- Bereitstellen der Empfehlungsinformation.

Das Verfahren zu einem Bereitstellen einer Empfehlungsinformation für zumindest ein Magnetresonanz-Protokoll für eine Magnetresonanzuntersuchung an einem Patienten ist dazu ausgebildet, einen medizinischen Bedienpersonal, beispielsweise einen Arzt und/oder Radiologen, bei der Auswahl eines Magnetresonanz-Protokolls für eine anstehende Magnetresonanzuntersuchung eines Patienten zu unterstützen. Dabei erhält der Benutzer eine Empfehlungsinformation für zumindest ein Magnetresonanz-Protokoll, wobei das ausgewählte oder empfohlene zumindest eine Magnetresonanz-Protokoll eine beste Übereinstimmung mit den Anforderungen, beispielsweise mit Patienteninformationen und/oder internen Arbeitsanweisungen, aufweist. Das Verfahren wird bevorzugt automatisiert ausgeführt. Hierzu muss ein medizinisches Bedienpersonal lediglich eine Planung der Magnetresonanzuntersuchung an dem Patienten starten und erhält dann automatisch die Empfehlungsinformation bereitgestellt.

Das Magnetresonanz-Protokoll umfasst bevorzugt mehrere Magnetresonanz-Sequenzen, wobei das Magnetresonanz-Protokoll eine definierte Reihenfolge einer Ausführung der mehreren Magnetresonanz-Sequenzen festlegt. Sofern für die anstehende Magnetresonanzuntersuchung Zwischenschritte, wie beispielsweise eine Kontrastmittelzugabe und/oder eine Neupositionierung des Patienten usw., benötigt werden, wird auch eine zeitliche Abfolge der Zwischenschritte zwischen den einzelnen Magnetresonanz-Sequenzen mittels des Magnetresonanz-Protokolls festlegt. Die einzelnen Magnetresonanz-Sequenzen sind bevorzugt auf eine klinische und/oder diagnostische Fragestellung abgestimmt, die mittels der Magnetresonanzuntersuchung am Patienten geklärt werden soll.

Die einzelnen Magnetresonanz-Sequenzen umfassen bevorzugt mehrere Hochfrequenzpulse und/oder mehrere Gradientenpulse, wobei die mehreren Hochfrequenzpulse und/oder die mehreren Gradientenpulse in einer definierten zeitlichen Reihenfolge ausgesendet werden, insbesondere in ein Field of View (FoV) einer Magnetresonanzvorrichtung eingestrahlt werden. Hierzu weist die Magnetresonanzvorrichtung, insbesondere eine medizinische Magnetresonanzvorrichtung, eine Hochfrequenzantenneneinheit und eine Gradientenspuleneinheit auf, die zu einem Aussenden der Hochfrequenzpulse und der Gradientenpulse ausgebildet sind.

Die untersuchungsrelevanten Patienteninformationen sind bevorzugt digital in zumindest einer Datenbank und/oder zumindest einer Speichereinheit hinterlegt, beispielsweise einer digitalen Patientendatenbank und/oder einem Radiologie-Informationssystem (RIS: Radiology Information System). Die untersuchungsrelevanten Patienteninformationen umfassen dabei eine aktuelle konkrete klinische und/oder diagnostische Fragestellung, die der Grund ist für die anstehende Magnetresonanzuntersuchung am Patienten. Bevorzugt umfassen die untersuchungsrelevanten Patienteninformationen alle zu dem Patienten gespeicherten Informationen und/oder Daten, die beispielsweise in einer Patientendatenbank und/oder einer RIS-Datenbank gespeichert sind.

Die zumindest eine untersuchungsrelevante Anwenderinformation umfasst bevorzugt eine untersuchungsrelevante Information eines Anwenders, wobei ein Anwender ein Arzt, ein Krankenhaus, eine Radiologie-Praxis usw. sein kann. Die untersuchungsrelevante Anwenderinformation kann beispielsweise eine lokale und/oder betreiberabhängige Vorschrift und/oder Information zur Magnetresonanzuntersuchung umfassen.

Das Bereitstellen der zumindest einen untersuchungsrelevanten Patienteninformationen und/oder der zumindest einer untersuchungsrelevanten Anwenderinformation erfolgt bevorzugt mittels einer Bereitstellungseinheit. Die Bereitstellungseinheit kann dabei von der Magnetresonanzvorrichtung umfasst sein. Zudem kann die Bereitstellungseinheit auch von der Datenbank, in der die zumindest eine untersuchungsrelevante Patienteninformation und/oder die zumindest eine untersuchungsrelevante Anwenderinformation gespeichert ist, umfasst sein. Zudem kann die Bereitstellungseinheit auch von einer Recheneinheit umfasst sein, wobei die Recheneinheit für die Planung der Magnetresonanzuntersuchung des Patienten von dem medizinischen Bedienpersonal verwendet wird und insbesondere zu einem Steuern des Verfahrens zu einem Bereitstellen einer Empfehlungsinformation für zumindest ein Magnetresonanz-Protokoll für eine Magnetresonanzuntersuchung an einem Patienten ausgebildet ist.

Das Bereitstellen der zumindest einen untersuchungsrelevanten Patienteninformation und/oder der zumindest einen untersuchungsrelevanten Anwenderinformation kann dabei auch ein Generieren einer Textzeichenfolge aus der zumindest einen untersuchungsrelevanten Patienteninformation und/oder der zumindest einen untersuchungsrelevanten Anwenderinformation umfassen, die für eine Analyse oder Bearbeitung durch ein trainiertes Sprachen-Modell bereitgestellt wird. Bevorzugt erfolgt das Generieren der Textzeichenfolge aus der zumindest einen untersuchungsrelevanten Patienteninformation und/oder der zumindest einen untersuchungsrelevanten Anwenderinformation durch die Recheneinheit.

Die Protokollinformation für ein Magnetresonanz-Protokoll kann im einfachsten Fall nur den Namen des Protokolls umfassen. Bevorzugterweise umfasst die Protokollinformation für ein Magnetresonanz-Protokoll zusätzliche Informationen zur dem Magnetresonanz-Protokoll, wie beispielsweise welche Magnetresonanz-Sequenzen in dem Magnetresonanz-Protokoll verwendet werden und/oder welche Eigenschaften dieses Magnetresonanz-Protokoll umfasst und/oder welche Einstellungen dieses Magnetresonanz-Protokoll aufweist usw.

Die Gesamtheit der zur Verfügung stehenden Magnetresonanz-Protokolle umfasst bevorzugt alle Magnetresonanz-Protokolle, die lokal und/oder vor Ort für eine Verwendung mit der ausgewählten und/oder der zur Verfügung stehenden Magnetresonanzvorrichtung in einer lokalen Datenbank gespeichert sind. Die Gesamtheit der zur Verfügung stehenden Magnetresonanz-Protokolle kann dabei in einem Protokollbaum gespeichert und/oder sortiert sein. Beispielsweise sind in einem Protokollbaum die einzelnen Magnetresonanz-Protokolle nach den zu untersuchenden Körperbereichen von Patienten sortiert. Zudem sind auch weitere, dem Fachmann als sinnvoll erscheinende Sortierung der Magnetresonanz-Protokolle jederzeit denkbar.

Das Bereitstellen von Protokollinformationen zu unterschiedlichen Magnetresonanz-Protokollen aus der Gesamtheit an zur Verfügung stehenden Magnetresonanz-Protokollen kann dabei auf diejenigen unterschiedlichen Magnetresonanz-Protokolle beschränkt sein, die den für die anstehende Magnetresonanzuntersuchung am Patienten relevanten Körperbereich umfassen. Bevorzugt ist die Gesamtheit der zur Verfügung stehender Magnetresonanz-Protokolle unterteilt in unterschiedliche Protokoll-Gruppen, wobei die unterschiedlichen Protokoll-Gruppen jeweils einem definierten Körperbereich zugeordnet sind. Bevorzugt weist eine Protokoll-Gruppe alle Magnetresonanz-Protokolle auf, die für eine Magnetresonanzuntersuchung dieses definierten Körperbereichs ausgebildet sind. Beispielsweise können für eine klinische und/oder diagnostische Fragestellung, die den Kopfbereich des Patienten betrifft, nur Magnetresonanz-Protokolle bereitgestellt werden, die der Protokoll-Gruppe "Kopf" zugeordnet und für Kopfuntersuchung ausgelegt und/oder ausgebildet sind. In einem anderen Beispiel können bei Knieproblemen des Patienten nur Magnetresonanz-Protokolle bereitgestellt werden, die der Protokoll-Gruppe "Knie" zugeordnet und für Knieuntersuchungen ausgelegt und/oder ausgebildet sind.

Das Bereitstellen der Protokollinformation zu unterschiedlichen Magnetresonanz-Protokollen aus einer Gesamtheit an zur Verfügung stehenden Magnetresonanz-Protokollen erfolgt bevorzugt mittels einer Bereitstellungseinheit. Die Bereitstellungseinheit kann dabei von der Magnetresonanzvorrichtung umfasst sein. Zudem kann die Bereitstellungseinheit auch von der Datenbank, in der die Gesamtheit an zur Verfügung stehenden Magnetresonanz-Protokolle gespeichert ist, umfasst sein. Zudem kann die Bereitstellungseinheit auch von einer Recheneinheit umfasst sein, wobei die Recheneinheit für die Planung der Magnetresonanzuntersuchung des Patienten von dem medizinischen Bedienpersonal verwendet wird und insbesondere zu einem Steuern des Verfahrens zu einem Bereitstellen einer Empfehlungsinformation für zumindest ein Magnetresonanz-Protokoll für eine Magnetresonanzuntersuchung an einem Patienten ausgebildet ist.

Das Bereitstellen der Protokollinformation zu unterschiedlichen Magnetresonanz-Protokollen aus der Gesamtheit an zur Verfügung stehenden Magnetresonanz-Protokollen kann dabei auch ein Generieren unterschiedlicher Textzeichenfolgen umfassen, wobei jede Textzeichenfolge genau einem Magnetresonanz-Protokoll zugeordnet ist. Die unterschiedlichen Textzeichenfolgen können anschließend für eine Analyse oder Bearbeitung durch das trainierte Sprachen-Modell bereitgestellt werden. Bevorzugt erfolgt das Generieren der Textzeichenfolgen für die Protokollinformationen der unterschiedlichen Magnetresonanz-Protokolle durch die Recheneinheit.

Das Sprachen-Modell (englisch: Language Model (LM)) ist ein mathematisches Modell, das die Abfolge von Elementen in einer Sequenz, beispielsweise von Buchstaben und/oder Wörtern, modelliert. Das Sprachen-Modell kann so konfiguriert sein, dass es natürliche Sprache und insbesondere einzelne Elemente, wie Wörter, in Eingaben, die natürliche Sprache enthalten, erkennt und/oder versteht und die Elemente in eine Textausgabe überträgt. Der natürliche Sprachverarbeitungsalgorithmus basiert bevorzugterweise auf einer trainierten oder maschinell erlernten Funktion. Alternativ kann der Algorithmus zur Verarbeitung natürlicher Sprache regelbasiert sein.

Besonders vorteilhaft umfasst das trainierte Sprachen-Modell ein trainiertes Großes Sprachen-Modell (englisch: Large Language Model (LLM)), insbesondere ein embedded LLM. Das trainierte LLM kann ein computerlinguistisches Wahrscheinlichkeitsmodell repräsentieren, das statistische Wort- und Satzfolge-Beziehungen aus einer Vielzahl von Textdokumenten durch einen rechenintensiven Trainingsprozess erlernt hat. Insbesondere werden die Wahrscheinlichkeiten des trainierten LLMs von künstlichen neuronalen Netzen berechnet.

Das trainierte LLM ist bevorzugt dazu ausgebildet und/oder trainiert, die untersuchungsrelevanten Patienteninformationen und/oder die untersuchungsrelevanten Anwenderinformationen zu analysieren und daraus einen Patienten-Merkmalsvektor zu generieren. Insbesondere ist das trainierte LLM dazu ausgebildet und/oder trainiert, die bereitgestellte, aus den untersuchungsrelevanten Patienteninformationen und/oder den untersuchungsrelevanten Anwenderinformationen generierte Textzeichenfolge zu analysieren und daraus einen Patienten-Merkmalsvektor zu generieren. Der Patienten-Merkmalsvektor fasst bevorzugt die parametrisierbaren Eigenschaften eines Musters, insbesondere der untersuchungsrelevanten Patienteninformationen und/oder der untersuchungsrelevanten Anwenderinformationen der Textzeichenfolge, in vektorieller Weise zusammen. Dabei können verschieden Merkmale, die für die untersuchungsrelevanten Patienteninformationen und/oder die untersuchungsrelevanten Anwenderinformationen charakteristisch sind, verschiedene Dimensionen des Patienten-Merkmalsvektor abbilden. Der Patienten-Merkmalsvektor umfasst eine Zahlenfolge, die die charakteristischen Merkmale der untersuchungsrelevanten Patienteninformationen und/oder der untersuchungsrelevanten Anwenderinformationen umfassen und/oder darstellen.

Bevorzugt ist das trainierte LLM weiterhin dazu ausgebildet und/oder trainiert, die Protokollinformationen für die unterschiedlichen Magnetresonanz-Protokolle zu analysieren und daraus einen Protokoll-Merkmalsvektor für jedes Magnetresonanz-Protokoll der unterschiedlichen Magnetresonanz-Protokolle zu generieren. Insbesondere ist das trainierte LLM dazu ausgebildet und/oder trainiert, die bereitgestellten, aus den Protokollinformationen generierten Textzeichenfolgen zu analysieren und daraus für jede Textzeichenfolge einen Protokoll-Merkmalsvektor zu generieren. Der Protokoll-Merkmalsvektor fasst bevorzugt die parametrisierbaren Eigenschaften eines Musters, insbesondere einer Textzeichenfolge einer Protokollinformation eines Magnetresonanz-Protokolls, in vektorieller Weise zusammen. Dabei können verschieden Merkmale, die für das Magnetresonanz-Protokoll charakteristisch sind, verschiedene Dimensionen des Protokoll-Merkmalsvektor abbilden. Der Protokoll-Merkmalsvektor umfasst eine Zahlenfolge, die die charakteristischen Merkmale des Magnetresonanz-Protokolls und/oder der Protokollinformation des Magnetresonanz-Protokolls darstellen. Für die unterschiedlichen Magnetresonanz-Protokolle werden somit unterschiedliche Protokoll-Merkmalsvektoren von dem trainierten LLM ermittelt und bereitgestellt. Insbesondere wird für jedes Magnetresonanz-Protokoll genau ein Protokoll-Merkmalsvektor generiert und bereitgestellt.

Das Anwenden des trainierten Sprachen-Modells, insbesondere des trainierten LLMs, auf die die zumindest eine untersuchungsrelevanten Patienteninformation und/oder die zumindest eine untersuchungsrelevanten Anwenderinformation wird von einer Recheneinheit durchgeführt, die hierzu das trainierte Sprachen-Modell, insbesondere das trainierte LLM, umfasst. Zudem wird auch das Anwenden des trainierten Sprachen-Modells, insbesondere des trainierten LLMs, auf die Protokollinformationen der unterschiedlichen Magnetresonanz-Protokolle mittels der Recheneinheit durchgeführt. Die Recheneinheit weist zudem eine Eingangsschnittstelle, die ein Zugreifen auf die zumindest eine untersuchungsrelevante Patienteninformation und/oder die zumindest eine untersuchungsrelevante Anwenderinformation ermöglicht. Die Eingangsschnittstelle der Recheneinheit ermöglicht weiterhin auch das Zugreifen auf die Protokollinformationen der unterschiedlichen Magnetresonanz-Protokolle.

Bevorzugt wird das trainierte Sprachen-Modell, insbesondere das trainierte LLM, sowohl für die Ermittlung und/oder Generierung des Patienten-Merkmalsvektors als auch für die Ermittlung und/oder Generierung der Protokoll-Merkmalsvektoren verwendet. Der Patienten-Merkmalsvektor und die mehreren Protokoll-Merkmalsvektoren werden von dem trainierten Sprachen-Modell für eine weiter Verarbeitung zur Ermittlung der Empfehlungsinformation bereitgestellt.

In einem weiteren Verfahrensschritt erfolgt ein Anwenden eines Ähnlichkeits-Algorithmus auf den Patienten-Merkmalsvektor und die unterschiedlichen Protokoll-Merkmalsvektoren. Mittels des Ähnlichkeits-Algorithmus wird ein Maß für eine Ähnlichkeit zwischen dem Patienten-Merkmalsvektor und den einzelnen Protokoll-Merkmalsvektoren ermittelt und damit ein Maß für eine Übereinstimmung zwischen dem Patienten-Merkmalsvektor und den einzelnen Protokoll-Merkmalsvektoren ermittelt. Derartige Ähnlichkeits-Algorithmen sind aus dem Stand der Technik bekannt. Beispielsweise kann der Ähnlichkeits-Algorithmus eine Kosinus-Ähnlichkeit umfassen, bei der ein Winkel zwischen zwei Vektoren, insbesondere dem Patienten-Merkmalsvektor und einem Protokoll-Merkmalsvektor, bestimmt wird. Ein Ähnlichkeitswert von 1 oder nahe 1 umfasst dabei einen Winkel von 0° oder nahe 0° zwischen den beiden Vektoren, insbesondere dem Patienten-Merkmalsvektor und einem Protokoll-Merkmalsvektor. Dieser Wert zeigt an, dass die beiden Vektoren, insbesondere der Patienten-Merkmalsvektor und ein Protokoll-Merkmalsvektor, nahezu identisch sind. Ein Ähnlichkeitswert von 0 oder nahe 0 zeigt an, dass die beiden Vektoren, insbesondere der Patienten-Merkmalsvektor und ein Protokoll-Merkmalsvektor, kaum eine Übereinstimmung aufweisen. Andere bekannte Beispiele für einen Ähnlichkeits-Algorithmus sind Euclidean distance (L2 Norm) oder Dot Product Similarity. Zudem sind weitere, dem Fachmann als sinnvoll erscheinende Ähnlichkeits-Algorithmen jederzeit denkbar.

Das Anwenden des Ähnlichkeits-Algorithmus erfolgt bevorzugt mittels der Recheneinheit, wobei die Recheneinheit zur Ermittlung der Empfehlungsinformation auf den von dem trainierten LLM bereitgestellten Patienten-Merkmalsvektor und die von dem trainierten LLM bereitgestellten unterschiedlichen Protokoll-Merkmalsvektoren zugreift. Die Empfehlungsinformation umfasst zumindest ein Magnetresonanz-Protokoll, wobei der Protokoll-Merkmalsvektor des zumindest einen Magnetresonanz-Protokolls der Empfehlungsinformation eine Maximal-Übereinstimmung zu dem Patienten-Merkmalsvektor aufweist. Die Maximal-Übereinstimmung umfasst die größte Übereinstimmung zwischen den unterschiedlichen Protokoll-Merkmalsvektoren mit dem Patienten-Merkmalsvektor.

Die Empfehlungsinformation kann nur das Magnetresonanz-Protokoll, dessen Protokoll-Merkmalsvektor die größte Übereinstimmung mit dem Patienten-Merkmalsvektor aufweist, aufweisen. Alternativ oder zusätzlich kann die Empfehlungsinformation auch eine Rankingliste aufweisen, in der die einzelnen Magnetresonanz-Protokolle in der Reihenfolge, mit der die Protokoll-Merkmalsvektoren der einzelnen Magnetresonanz-Protokolle mit dem Patienten-Merkmalsvektor übereinstimmen, aufgelistet sind.

Zudem wird die Empfehlungsinformation bereitgestellt. Die Bereitstellung der Empfehlungsinformation erfolgt bevorzugt mittels einer Bereitstellungsschnittstelle und/oder einer Ausgangsschnittstelle der Recheneinheit. Bevorzugt wird die Empfehlungsinformation für eine Ausgabe, insbesondere eine visuelle Ausgabe, an das medizinische Bedienpersonal bereitgestellt. Die Ausgabe der Empfehlungsinformation erfolgt bevorzugt mittels einer Ausgabeeinheit, insbesondere einer visuellen Ausgabeeinheit, wie beispielweise einem Monitor und/oder einem Display, einer Benutzerschnittstelle.

Durch die Erfindung kann vorteilhaft eine schnelle Empfehlung für zumindest ein Magnetresonanz-Protokoll einem medizinischen Bedienpersonal bei der Planung der Magnetresonanzuntersuchung bereitgestellt werden. Insbesondere kann durch die Erfindung sichergestellt werden, dass alle über den Patienten zur Verfügung stehenden Informationen bei der Ermittlung der Empfehlungsinformation und damit auch bei der Planung der Magnetresonanzuntersuchung berücksichtigt werden können. Ein weiterer Vorteil ist, dass der Empfehlungsinformation eine neutrale Analyse für die unterschiedlichen Magnetresonanz-Protokolle zugrunde liegt, um die beste Übereinstimmung zwischen den Untersuchungsanforderungen und den einzelnen Magnetresonanz-Protokollen zu ermitteln. Insbesondere können dabei auch Magnetresonanz-Protokolle, die aufgrund einer Vorliebe des medizinischen Bedienpersonals und/oder aufgrund eines aufwändigen Workflows bei einer manuellen Auswahl kaum oder nie berücksichtigt werden, neutral bewertet werden und empfohlen werden.

Durch die Erfindung kann ein Verfahren bereitgestellt werden, das Informationen aus unterschiedlichen Quellen und/oder Informationen in unterschiedlichen Formaten bei der Ermittlung der Empfehlungsinformation berücksichtigt. Auch werden stets alle aktuellen Magnetresonanz-Protokolle und auch neue Protokoll-Varianten eines Magnetresonanz-Protokolls bei der Ermittlung der Empfehlungsinformation berücksichtigt. Ein weiterer Vorteil der Erfindung ist, dass mittels eines trainierten LLMs ein Instrument bereitgestellt wird, mittels dessen eine hohe Geschwindigkeit und/oder eine hohe Effizient für eine Bereitstellen einer Empfehlungsinformation für die Magnetresonanzuntersuchung vorteilhaft erreicht werden kann.

In einer vorteilhaften Weiterbildung des erfindungsgemäßen Verfahrens kann es vorgesehen sein, dass die zumindest eine untersuchungsrelevante Patienteninformation zumindest eine der folgenden Informationen umfasst:
- Daten und/oder Informationen einer vorhergehenden Untersuchung des Patienten und/oder eines bisherigen Krankheitsverlaufs des Patienten,
- Daten und/oder Informationen, die von einem medizinischen Bedienpersonal in Vorbereitung zu der anstehenden Magnetresonanzuntersuchung des Patienten erstellt wurden, und/oder
- Notizen und/oder Bemerkungen, die von einem medizinischen Bedienpersonal zu dem Patienten erstellt wurden.

Die untersuchungsrelevanten Patienteninformationen umfassen bevorzugt digitale Patienteninformationen, die in einer Datenbank, insbesondere einer Patientendatenbank und/oder RIS-Datenbank, gespeichert sind. Insbesondere umfassen die untersuchungsrelevanten Patienteninformationen alle in einer Datenbank oder auch in mehreren Datenbanken hinterlegten Patienteninformationen.

Die vorhergehende Untersuchung des Patienten kann beispielsweise eine Voruntersuchung zu der anstehenden Magnetresonanzuntersuchung sein. Zudem kann die vorhergehenden Untersuchung auch eine Untersuchung zu einer klinischen und/oder diagnostischen Fragestellung umfassen, die zu der klinischen und/oder diagnostischen Fragestellung der anstehenden Magnetresonanzuntersuchung unterschiedlich ausgebildet ist.

Die Notizen und/oder Bemerkungen, die von einem medizinischen Personal, beispielsweise einem Arzt und/oder einem Radiologen, zu dem Patienten erstellt wurden, können beispielsweise einen Arztbrief und/oder weitere Bemerkungen umfassen. Des Weiteren können die untersuchungsrelevanten Patienteninformationen auch weitere Patientendaten, wie beispielsweise ein Alter des Patienten, eine Größe und/oder Gewicht des Patienten usw., umfassen.

Diese Ausgestaltung der Erfindung weist den Vorteil auf, dass alle zur Verfügung stehende Informationen und/oder Daten des Patienten, die in einer Datenbank gespeichert sind, bei der Ermittlung der Empfehlungsinformation für die anstehende Magnetresonanzuntersuchung berücksichtigt werden. Beispielsweise kann ein Patient eine Erkrankung und/oder Beeinträchtigung aufweisen, die nur eine kurze Aufenthaltsdauer in einer Magnetresonanzvorrichtung erlaubt, jedoch unabhängig ist zu der Ursache für die anstehende Magnetresonanzuntersuchung. Durch die Berücksichtigung aller Informationen des Patienten kann somit die Empfehlungsinformation ein Magnetresonanz-Protokoll umfassen, das nur eine kurze Messzeit aufweist.

In einer vorteilhaften Weiterbildung des erfindungsgemäßen Verfahrens kann es vorgesehen sein, dass die zumindest eine untersuchungsrelevante Anwenderinformation eine betreiberabhängige und/oder lokale Anweisung und/oder Vorschrift zur Magnetresonanzuntersuchung umfasst. Die eine betreiberabhängige und/oder lokale Anweisung und/oder Vorschrift kann beispielsweise eine krankenhausinterne Anweisung zum Ausführen bestimmter Magnetresonanzuntersuchungen umfassen. Zudem kann die betreiberabhängige und/oder lokale Anweisung und/oder Vorschrift auch länderabhängige Regelungen zum Ausführen bestimmter Magnetresonanzuntersuchungen umfassen. Derart können vorteilhaft betreiberabhängige Informationen und/oder lokale Anwenderinformationen bei der Ermittlung der Empfehlungsinformation berücksichtigt werden.

In einer vorteilhaften Weiterbildung des erfindungsgemäßen Verfahrens kann es vorgesehen sein, dass die zumindest eine untersuchungsrelevante Patienteninformation und/oder die zumindest eine untersuchungsrelevante Anwenderinformation einen Freitext umfasst. Der Freitext umfasst keine Bedingungen an eine definierte Datenstruktur und/oder eine definiert Textstruktur und/oder ein definiertes Datenformat des vorliegenden Textes. Auch ist es möglich, dass der Freitext Tippfehler und/oder Rechtschreibfehler und/oder Grammatikfehler aufweisen kann. Hierdurch kann ein einfaches und schnelles Bereitstellen der zumindest einen untersuchungsrelevanten Patienteninformation und/oder der zumindest einen untersuchungsrelevanten Anwenderinformation ermöglicht werden. Insbesondere kann derart auf eine zeitaufwendige Formatierung der zumindest einen untersuchungsrelevanten Patienteninformation und/oder der zumindest einen untersuchungsrelevanten Anwenderinformation vor einem Bereitstellen der zumindest einen untersuchungsrelevanten Patienteninformation und/oder der zumindest einen untersuchungsrelevanten Anwenderinformation für eine Ermittlung der Empfehlungsinformation vorteilhaft verzichtet werden.

In einer vorteilhaften Weiterbildung des erfindungsgemäßen Verfahrens kann es vorgesehen sein, dass das Bereitstellen der zumindest einen untersuchungsrelevanten Patienteninformation und/oder der zumindest einen untersuchungsrelevanten Anwenderinformation ein Generieren einer Textzeichenfolge aus der zumindest einen untersuchungsrelevanten Patienteninformation und/oder der zumindest einen untersuchungsrelevanten Anwenderinformation umfasst. Insbesondere umfasst das Generieren einer Textzeichenfolge dabei ein Generieren einer einzigen Textzeichenfolge für alle zur Verfügung stehenden untersuchungsrelevanten Patienteninformationen und zur Verfügung stehenden untersuchungsrelevanten Anwenderinformationen. Die Textzeichenfolge umfasst bevorzugt eine Zeichenkette und/oder einen String aus Textzeichen. Diese Ausgestaltung der Erfindung ermöglicht eine einfache und direkte Informationsgenerierung durch ein trainiertes Sprachen-Modell, insbesondere ein LLM.

In einer vorteilhaften Weiterbildung des erfindungsgemäßen Verfahrens kann es vorgesehen sein, dass das Bereitstellen von Protokollinformationen zu unterschiedlichen Magnetresonanz-Protokollen aus der Gesamtheit an zur Verfügung stehenden Magnetresonanz-Protokollen eine Beschränkung von Protokollinformationen zu unterschiedlichen Magnetresonanz-Protokollen einer Protokoll-Gruppe umfasst. Die Gesamtheit der zur Verfügung stehenden Magnetresonanz-Protokollen umfasst bevorzugt mehrere Protokoll-Gruppen. Jede der mehreren Protokoll-Gruppen ist bevorzugt auf Magnetresonanz-Protokolle eines definierten Untersuchungsbereichs beschränkt. Der definierte Untersuchungsbereich kann beispielweise den Kopf oder das Knie oder das Herz usw. umfassen. Das Bereitstellen von Protokollinformationen zu unterschiedlichen Magnetresonanz-Protokollen kann dabei eine Bereitstellen von Protokollinformationen zu allen Magnetresonanz-Protokollen, die einer Protokoll-Gruppe zugeordnet sind, umfassen. Hierdurch können vorteilhaft alle für die anstehende Magnetresonanzuntersuchung relevanten Magnetresonanz-Protokolle bei der Ermittlung der Empfehlungsinformation berücksichtigt werden.

Die Auswahl einer Protokoll-Gruppe kann dabei automatisch durch die Recheneinheit erfolgen, beispielsweise anhand der untersuchungsrelevanten Patienteninformation. Zudem kann die Auswahl einer Protokoll-Gruppe auch durch ein medizinisches Bedienpersonal erfolgen.

In einer vorteilhaften Weiterbildung des erfindungsgemäßen Verfahrens kann es vorgesehen sein, dass das das Bereitstellen von Protokollinformationen zu unterschiedlichen Magnetresonanz-Protokollen ein Generieren einer Textzeichenfolge aus jeder Protokollinformation eines Magnetresonanz-Protokolls umfasst. Mit anderen Worten wird dabei für jedes Magnetresonanz-Protokoll der unterschiedlichen Magnetresonanz-Protokolle anhand der jeweiligen Protokollinformation eine Textzeichenfolge generiert, so dass für jedes der unterschiedlichen Magnetresonanz-Protokolle eine genierte Textzeichenfolge vorliegt. Diese Ausgestaltung der Erfindung ermöglicht eine einfache und direkte Informationsgenerierung durch ein trainiertes Sprachen-Modell, insbesondere ein LLM.

In einer vorteilhaften Weiterbildung des erfindungsgemäßen Verfahrens kann es vorgesehen sein, dass das trainierte Sprachen-Modell, insbesondere das trainierte LLM, ein auf einem neuronalen Netzwerk basierendes Encoder-Netzwerk umfasst, das zu einer Kodierung der Textzeichenfolgen ausgebildet ist. Das Encoder-Netzwerk ist bevorzugt dazu trainiert, Informationen aus den jeweiligen Textzeichenfolgen der zumindest einen untersuchungsrelevanten Patienteninformation und/oder der zumindest einen untersuchungsrelevanten Anwenderinformation und/oder der Protokollinformationen zu kodieren. Diese Kodierung wird zur Ermittlung des Patienten-Merkmalsvektors und/oder der Protokoll-Vektoren verwendet. Dabei umfasst der Patienten-Merkmalsvektor die Kodierung der zumindest einen untersuchungsrelevanten Patienteninformation und/oder der zumindest einen untersuchungsrelevanten Anwenderinformation. Die Protokoll-Merkmalsvektoren umfassen die Kodierung der Protokollinformationen. Die Kodierung ermöglicht einen einfachen und schnellen Vergleich zwischen den Patienten-Merkmalsvektoren und den unterschiedlichen Protokoll-Merkmalsvektoren.

In einer vorteilhaften Weiterbildung des erfindungsgemäßen Verfahrens kann es vorgesehen sein, dass die Empfehlungsinformation mehrere Magnetresonanz-Protokolle umfasst, wobei die mehreren Magnetresonanz-Protokolle hinsichtlich einer Übereinstimmung zwischen dem Patienten-Merkmalsvektor und dem Protokoll-Merkmalsvektor des jeweiligen Magnetresonanz-Protokolls sortiert sind. Bevorzugt sind hierbei die mehreren Magnetresonanz-Protokolle derart sortiert, dass die Magnetresonanz-Protokolle mit der größten Übereinstimmung mit dem Patienten-Merkmalsvektor zuerst aufgeführt sind. Hierdurch kann ein Benutzer, insbesondere das medizinische Bedienpersonal, eine Empfehlungsinformation erhalten, die eine vorteilhafte Übersicht über die mehreren Magnetresonanz-Protokolle und deren Übereinstimmung mit den Anforderung an die Magnetresonanzuntersuchung an dem Patienten enthält. Damit kann das medizinischen Bedienpersonal bei einer Auswahl eines Magnetresonanz-Protokolls für die anstehende Magnetresonanzuntersuchung vorteilhaft unterstützt werden.

Auch kann die Empfehlungsinformation einen Wert einer Übereinstimmung zwischen dem Patienten-Merkmalsvektor und den Protokoll-Merkmalsvektoren für die mehreren Magnetresonanz-Protokolle umfassen. Dies ermöglicht zusätzlich, dass das medizinische Bedienpersonal anhand des Werts der Übereinstimmung des jeweiligen Magnetresonanz-Protokolls und beispielsweise einer persönlichen Erfahrung im Umgang und/oder der Ausführung des Magnetresonanz-Protokolls ein geeignetes Magnetresonanz-Protokolls auswählen kann.

In einer vorteilhaften Weiterbildung des erfindungsgemäßen Verfahrens kann es vorgesehen sein, dass in einem weiteren Verfahrensschritt eine Ausgabe der bereitgestellten Empfehlungsinformation an einen Benutzer erfolgt. Vorzugsweise erfolgt die Ausgabe mittels einer Ausgabeeinheit, insbesondere einer visuellen Ausgabeeinheit, wie beispielsweise ein Monitor und/oder eine Display. Die Ausgabeeinheit ist bevorzugt von einer Benutzerschnittstelle umfasst. Die Benutzerschnittstelle kann dabei von der Magnetresonanzvorrichtung umfasst sein. Zudem ist es auch denkbar, dass die Benutzerschnittstelle von einer Planungseinheit, die zu einer Planung der Magnetresonanzuntersuchung ausgebildet ist, oder der Recheneinheit umfasst ist. Derart kann eine vorteilhafte Übermittlung der Empfehlungsinformation an den Benutzer erreicht werden.

In einer vorteilhaften Weiterbildung des erfindungsgemäßen Verfahrens kann es vorgesehen sein, dass die Ausgabe der Empfehlungsinformation eine Voreinstellung einer Protokollauswahl bei einer Planung der Magnetresonanzuntersuchung des Patienten umfasst. Derart kann eine einfache Übernahme der Empfehlungsinformation für die Magnetresonanzuntersuchung des Patienten erreicht werden. Bei einer Übernahme der Empfehlung kann das medizinische Bedienpersonal die Vorauswahl einfach bestätigen und das empfohlene Magnetresonanz-Protokoll ist ausgewählt. Zudem hat derart das medizinische Bedienpersonal weiterhin die Möglichkeit, die Empfehlung abzulehnen und eine andere Protokollauswahl zu tätigen.

In einer vorteilhaften Weiterbildung des erfindungsgemäßen Verfahrens kann es vorgesehen sein, dass das Sprachen-Modell, insbesondere das LLM, anhand der ermittelten Empfehlungsinformation nachtrainiert wird. Derart kann das Sprachen-Modell, insbesondere das LLM, auch angepasst werden. Beispielsweise können durch eine Anpassung des Sprachen-Modells, insbesondere des LLMs, Magnetresonanz-Protokolle mit einer großen Übereinstimmung zu definierten Untersuchungsanforderungen bei einem erneuten Durchführen des Verfahrens einen höheren Übereinstimmungswert aufweisen. Dagegen können Magnetresonanz-Protokolle mit einer geringen Übereinstimmung zu definierten Untersuchungsanforderungen einen niedrigeren Übereinstimmungswert bei einem erneuten Durchführen des Verfahrens aufweisen.

Auch kann derart ein Benutzerfeedback zu einer ermittelten Empfehlungsinformation in das trainierte Sprachen-Modell, insbesondere das trainierte LLM, eingehen. Beispielsweise kann derart ein medizinisches Bedienpersonal angeben, ob die Empfehlungsinformation hilfreich war oder ob die empfohlenen Magnetresonanz-Protokolle auch zur Klärung der klinischen und/oder diagnostischen Fragestellung beigetragen haben.

Die Erfindung geht des Weiteren aus von einer Recheneinheit zu einem Regeln und/oder Steuern eines Verfahrens zu einem Bereitstellen einer Empfehlungsinformation für zumindest ein Magnetresonanz-Protokoll für eine Magnetresonanzuntersuchung an einem Patienten, wobei die Recheneinheit umfasst:
- eine Eingangsschnittstelle, die dazu ausgebildet ist, auf zumindest eine Datenbank zuzugreifen, in der die zumindest eine untersuchungsrelevante Patienteninformation und/oder die zumindest eine untersuchungsrelevante Anwenderinformation und/oder die Protokollinformationen zu unterschiedlichen Magnetresonanz-Protokollen gespeichert sind, um die zumindest eine untersuchungsrelevante Patienteninformation und/oder die zumindest eine untersuchungsrelevante Anwenderinformation und/oder die Protokollinformationen zu empfangen,
- ein Analysemodul, das dazu ausgebildet ist, mittels eines trainierten Sprachen-Modells einen Patienten-Merkmalsvektor anhand der zumindest einen untersuchungsrelevanten Patienteninformation und/oder der zumindest einer untersuchungsrelevanten Anwenderinformation bereitzustellen und mittels des trainierten Sprachen-Modells für die Protokollinformationen eines jeden Magnetresonanz-Protokolls einen Protokoll-Merkmalsvektor bereitzustellen, wobei das Analysemodul weiterhin dazu ausgebildet ist, mittels eines Ähnlichkeits-Algorithmus eine Empfehlungsinformation anhand des Patienten-Merkmalsvektors und der Protokoll-Merkmalsvektoren zu ermitteln, und
- eine Ausgangsschnittstelle, die zu einem Bereitstellen der Empfehlungsinformation ausgebildet ist.

Die Recheneinheit kann dabei einen Prozessor aufweisen, der von dem Analysemodul umfasst ist. Die Recheneinheit kann zudem eine zentrale Recheneinheit und/oder ein "Edge Device" sein. Die Recheneinheit kann zum Ausführen des Verfahrens gemäß dem Verfahrensaspekt ausgebildet sein. Alternativ oder ergänzend kann die Recheneinheit jedes beliebige Merkmal umfassen, das im Kontext des Verfahrens gemäß dem Verfahrensaspekt offenbart ist.

Die Recheneinheit kann als Datenverarbeitungssystem oder als Teil eines Datenverarbeitungssystems realisiert sein. Ein solches Datenverarbeitungssystem kann beispielsweise ein Cloud-Computing-System, ein Computernetzwerk, einen Computer, einen Tablet-Computer, ein Smartphone und/oder dergleichen umfassen. Die Recheneinheit kann aus Hardware und/oder Software bestehen. Die Hardware kann beispielsweise einen oder mehrere Prozessoren, einen oder mehrere Speicher und Kombinationen davon umfassen. Der eine oder mehrere Speicher können Anweisungen zum Ausführen der erfindungsgemäßen Verfahrensschritte speichern. Die Hardware kann von der Software konfigurierbar und/oder von der Software bedienbar sein. Generell können alle Einheiten, Untereinheiten oder Module zumindest vorübergehend miteinander im Datenaustausch stehen, z.B. über eine Netzwerkverbindung oder entsprechende Schnittstellen. Folglich können sich einzelne Einheiten auch voneinander entfernt befinden.

Bevorzugt weist die Recheneinheit eine Schnittstelleneinheit auf, die die Eingangsschnittstelle und die Ausgangsschnittstelle aufweist. Die Schnittstelleneinheit kann eine Schnittstelle zum Datenaustausch mit einem lokalen Server oder einen zentralen Webserver über eine Internetverbindung zum Empfangen von Daten und/oder Informationen umfassen. Die Schnittstelleneinheit kann ferner angepasst werden, um mit einem oder mehreren Benutzern des Systems zu kommunizieren, z.B. indem sie dem Benutzer das Ergebnis der Verarbeitung durch die Recheneinheit anzeigt (z.B. in einer grafischen Benutzerschnittstelle) oder indem sie dem Benutzer ermöglicht, Parameter für die Datenverarbeitung oder Visualisierung anzupassen. Mit anderen Worten, die Schnittstelleneinheit kann eine Benutzerschnittstelle umfassen.

Die Vorteile der erfindungsgemäßen Recheneinheit entsprechen im Wesentlichen den Vorteilen des erfindungsgemäßen Verfahrens zu einem Bereitstellen einer Empfehlungsinformation für zumindest ein Magnetresonanz-Protokoll für eine Magnetresonanzuntersuchung, welche vorab im Detail ausgeführt sind. Hierbei erwähnte Merkmale, Vorteile oder alternative Ausführungsformen können ebenso auf die anderen beanspruchten Gegenstände übertragen werden und umgekehrt.

Weiterhin geht die Erfindung aus von einem System zu einem Bereitstellen einer Empfehlung zumindest eines Magnetresonanz-Protokoll für eine Magnetresonanzuntersuchung an einem Patienten, umfassend:
- eine Recheneinheit, die zu einem Regeln und/oder Steuern eines Verfahrens zu einem Bereitstellen einer Empfehlungsinformation für zumindest ein Magnetresonanz-Protokoll für eine Magnetresonanzuntersuchung an einem Patienten ausgebildet ist,
- zumindest eine digitale Datenbank, in der die zumindest eine untersuchungsrelevante Patienteninformation und/oder die zumindest eine untersuchungsrelevante Anwenderinformation und/oder die Protokollinformationen gespeichert sind.

Bevorzugt umfasst das System zwei oder mehr digitale Datenbanken zur Speicherung der zumindest einen untersuchungsrelevanten Patienteninformation, der untersuchungsrelevanten Anwenderinformation und den Patienteninformationen. Eine erste digitale Datenbank des Systems, beispielsweise eine digitale Patientendatenbank oder eine RIS-Datenbank, ist zur Speicherung der untersuchungsrelevanten Patienteninformationen ausgebildet. Die untersuchungsrelevante Anwenderinformation ist bevorzugt in einer zweiten digitalen Datenbank des Systems gespeichert. Die Protokollinformationen sind bevorzugt in einer weiteren, dritten digitalen Datenbank gespeichert. Zudem kann es auch sein, dass die untersuchungsrelevanten Anwenderinformationen und die Protokollinformationen in einer gemeinsamen digitalen Datenbank gespeichert sind.

Die Vorteile des erfindungsgemäßen Systems entsprechen im Wesentlichen den Vorteilen des erfindungsgemäßen Verfahrens zu einem Bereitstellen einer Empfehlungsinformation für zumindest ein Magnetresonanz-Protokoll für eine Magnetresonanzuntersuchung, welche vorab im Detail ausgeführt sind. Hierbei erwähnte Merkmale, Vorteile oder alternative Ausführungsformen können ebenso auf die anderen beanspruchten Gegenstände übertragen werden und umgekehrt.

Gemäß einem weiteren Aspekt der Erfindung wird ein Computerprogrammprodukt bereitgestellt mit Programmelementen, die eine Recheneinheit veranlassen, die Schritte des Verfahrens zu einem Bereitstellen einer Empfehlungsinformation für zumindest ein Magnetresonanz-Protokoll für eine Magnetresonanzuntersuchung an einem Patienten gemäß dem Verfahrensaspekt auszuführen, wenn die Programmelemente in einen Speicher der Recheneinheit geladen werden. Weitere Vorteile, Merkmale und Einzelheiten der Erfindung ergeben sich aus dem im Folgenden beschriebenen Ausführungsbeispiel sowie anhand der Zeichnungen.

Es zeigen:
- Fig. 1: ein Ablaufdiagramm eines erfindungsgemäßes Verfahrens zu einem Bereitstellen einer Empfehlungsinformation für zumindest ein Magnetresonanz-Protokoll für eine Magnetresonanzuntersuchung,
- Fig. 2: ein Datenflussdiagramm des Verfahrens zu einem Bereitstellen einer Empfehlungsinformation für zumindest ein Magnetresonanz-Protokoll für eine Magnetresonanzuntersuchung,
- Fig. 3: eine schematische Darstellung einer Recheneinheit zu einem Regeln und/oder Steuern des Verfahrens, und
- Fig. 4: ein System mit der Recheneinheit.

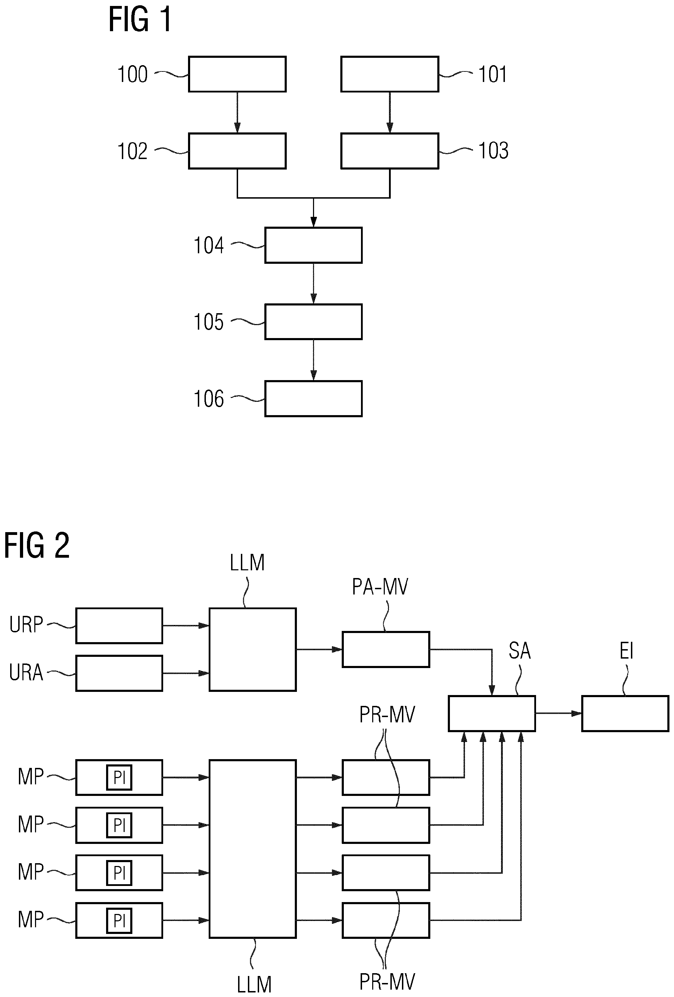

In den Fig. 1 und 2 ist ein erfindungsgemäßes Verfahren zu einem Bereitstellen einer Empfehlungsinformation EI für zumindest ein Magnetresonanz-Protokoll MP für eine Magnetresonanzuntersuchung an einem Patienten dargestellt. Das Verfahren wird von einer Recheneinheit 10 gesteuert.

In einem ersten Verfahrensschritt 100 erfolgt ein Bereitstellen von zumindest einer untersuchungsrelevanten Patienteninformation URP und/oder zumindest einer untersuchungsrelevanten Anwenderinformation URA. Das Bereitstellen erfolgt durch eine Bereitstellungseinheit. Die Bereitstellungseinheit ist bevorzugt von der Recheneinheit 10, insbesondere einer Eingangsschnittstelle 11 der Recheneinheit 10, umfasst. Alternativ oder zusätzlich kann die Bereitstellungseinheit auch von einer Datenbank, in der die zumindest eine untersuchungsrelevante Patienteninformation URP und/oder die zumindest eine untersuchungsrelevante Anwenderinformation URA gespeichert ist, umfasst sein.

Bevorzugt umfassen die untersuchungsrelevanten Patienteninformationen URP alle zur Verfügung stehenden Patienteninformationen. Die untersuchungsrelevante Patienteninformation URP kann dabei Daten und/oder Informationen einer vorhergehenden Untersuchung des Patienten und/oder eines bisherigen Krankheitsverlauf des Patienten umfassen. Alternativ oder zusätzlich kann die untersuchungsrelevante Patienteninformation URP auch Daten und/oder Informationen, die von einem medizinischen Personal in Vorbereitung zu der anstehenden Magnetresonanzuntersuchung des Patienten erstellt wurden, umfassen. Alternativ oder zusätzlich kann die untersuchungsrelevante Patienteninformation URP auch Notizen und/oder Bemerkungen, die von einem medizinischen Personal zu dem Patienten erstellt wurden, umfassen. Des Weiteren können die untersuchungsrelevanten Patienteninformationen URP auch weitere Patientendaten, wie beispielsweise ein Alter des Patienten, eine Größe und/oder Gewicht des Patienten, umfassen.

Die untersuchungsrelevante Anwenderinformation URA kann dabei eine untersuchungsrelevante Information eines Anwenders umfassen, wobei ein Anwender ein Arzt, ein Krankenhaus, eine Radiologie-Praxis usw. sein kann. Die untersuchungsrelevante Anwenderinformation URA kann beispielsweise eine lokale und/oder betreiberabhängige Vorschrift und/oder Information zu einer Magnetresonanzuntersuchung umfassen. Die eine betreiberabhängige und/oder lokale Anweisung und/oder Vorschrift kann beispielsweise eine krankenhausinterne Anweisung zum Ausführen bestimmter Magnetresonanzuntersuchungen umfassen. Zudem kann die betreiberabhängige und/oder lokale Anweisung und/oder Vorschrift auch länderabhängige Regelungen zum Ausführen bestimmter Magnetresonanzuntersuchungen umfassen.

Die zumindest eine untersuchungsrelevante Patienteninformation URP und/oder die zumindest eine untersuchungsrelevante Anwenderinformation URA sind bevorzugt als Freitext formuliert und/oder gespeichert. Das Bereitstellen der zumindest einen untersuchungsrelevanten Patienteninformation URP und/oder der zumindest einen untersuchungsrelevanten Anwenderinformation URA umfasst zudem ein Generieren einer Textzeichenfolge aus der zumindest einen untersuchungsrelevanten Patienteninformation URP und/oder der zumindest einen untersuchungsrelevanten Anwenderinformation URA.

In einem weiteren, zweiten Verfahrensschritt 101 erfolgt ein Bereitstellen von Protokollinformationen PI zu unterschiedlichen Magnetresonanz-Protokollen MP aus einer Gesamtheit an zur Verfügung stehenden Magnetresonanz-Protokollen MP. Das Bereitstellen erfolgt durch die Eingangsschnittstelle 11 der Recheneinheit 10, insbesondere durch die Bereitstellungseinheit. Dieser zweite Verfahrensschritt 101 kann dabei zeitgleich zu dem ersten Verfahrensschritt 100 ausgeführt werden.

Die Protokollinformation PI für ein Magnetresonanz-Protokoll MP kann im einfachsten Fall nur den Namen des Magnetresonanz-Protokolls MP umfassen. Bevorzugterweise umfasst die Protokollinformation PI für ein Magnetresonanz-Protokolls MP zusätzliche Informationen zur dem Magnetresonanz-Protokoll MP, wie beispielsweise welche Magnetresonanz-Sequenzen in dem Magnetresonanz-Protokoll MP verwendet werden und/oder welche Eigenschaften dieses Magnetresonanz-Protokoll MP umfasst usw.

Das Bereitstellen von Protokollinformationen PI zu unterschiedlichen Magnetresonanz-Protokollen MP aus der Gesamtheit an zur Verfügung stehenden Magnetresonanz-Protokollen MP umfasst bevorzugt eine Beschränkung von Protokollinformationen Pl zu unterschiedlichen Magnetresonanz-Protokollen MP einer Protokoll-Gruppe. Bevorzugt ist die Gesamtheit der zur Verfügung stehender Magnetresonanz-Protokolle MP unterteilt in unterschiedliche Protokoll-Gruppen, wobei die unterschiedlichen Protokoll-Gruppen jeweils einem definierten Körperbereich zugeordnet sind und bevorzugt alle Magnetresonanz-Protokolle MP dieses definierten Körperbereichs umfassen. Somit können die bereitgestellten Protokollinformationen Pl auf diejenigen Magnetresonanz-Protokolle MP beschränkt werden, die für Messungen an dem zu untersuchenden relevanten Körperbereich des Patienten ausgebildet sind.

Beispielsweise können für eine klinische und/oder diagnostische Fragestellung, die den Kopfbereich des Patienten betrifft, nur Magnetresonanz-Protokolle MP bereitgestellt werden, die der Protokoll-Gruppe "Kopf" zugeordnet und für Kopfuntersuchung ausgelegt und/oder ausgebildet sind. In einem anderen Beispiel können bei Knieproblemen des Patienten nur Magnetresonanz-Protokolle MP bereitgestellt werden, die der Protokoll-Gruppe "Knie" zugeordnet und für Knieuntersuchungen ausgelegt und/oder ausgebildet sind.

Die Auswahl einer Protokoll-Gruppe kann dabei automatisch durch die Recheneinheit 10 erfolgen, beispielsweise anhand der untersuchungsrelevanten Patienteninformation URP. Zudem kann die Auswahl einer Protokoll-Gruppe auch durch ein medizinisches Bedienpersonal erfolgen.

Das Bereitstellen der unterschiedlichen Protokollinformationen Pl umfasst zudem ein Generieren einer Textzeichenfolge. Dabei wird für jede bereitgestellte Protokollinformation PI eines Magnetresonanz-Protokolls MP eine eigene Textzeichenfolge generiert.

In einem weiteren, dritten Verfahrensschritt 102 erfolgt ein Anwenden eines trainierten Sprachen-Modells LLMs auf die bereitgestellten untersuchungsrelevanten Patienteninformationen URP und/oder die bereitgestellten untersuchungsrelevanten Anwenderinformationen URA, insbesondere auf die aus den untersuchungsrelevanten Patienteninformationen URP und/oder den untersuchungsrelevanten Anwenderinformationen URA generierte Textzeichenfolge. Das trainierte Sprachen-Modell umfasst dabei ein trainierte großes Sprachen-Modell LLM. Mittels des LLMs wird aus der Textzeichenfolge ein Patienten-Merkmalsvektor PA-MV ermittelt und bereitgestellt. Dabei umfasst das LLM ein auf einen neuronalen Netz basierendes Encoder-Netzwerk, das zu einer Kodierung der Textzeichenfolge ausgebildet ist und diese Kodierung in dem Patienten-Merkmalsvektor PA-MV bereitstellt.

In einem vierten Verfahrensschritt 103 erfolgt ein Anwenden des LLMs auf die bereitgestellten Protokollinformationen Pl der unterschiedlichen Magnetresonanz-Protokolle MP, insbesondere auf den aus den Protokollinformationen Pl generierten Textzeichenfolgen der unterschiedlichen Magnetresonanz-Protokolle MP. Mittels des LLMs wird aus der Textzeichenfolge ein Protokoll-Merkmalsvektor PR-MV für jedes der unterschiedlichen Magnetresonanz-Protokolle MP ermittelt und bereitgestellt. Insbesondere wird dabei mittels des LLMs eine Kodierung der Textzeichenfolgen ermittelt und diese Kodierung in dem Protokoll-Merkmalsvektor PR-MV bereitgestellt.

Der dritte Verfahrensschritt 102 und der vierte Verfahrensschritt 103 können dabei zeitgleich ausgeführt werden.

In einem weiteren, fünften Verfahrensschritt 104 erfolgt ein Anwenden eines Ähnlichkeits-Algorithmus SA auf den Patienten-Merkmalsvektor PA-MV und den unterschiedlichen Protokoll-Merkmalsvektoren PR-MV. Mittels des Ähnlichkeits-Algorithmus SA wird ein Maß für eine Ähnlichkeit zwischen dem Patienten-Merkmalsvektor PA-MV und den einzelnen Protokoll-Merkmalsvektoren PR-MV ermittelt und damit eine Übereinstimmung zwischen dem Patienten-Merkmalsvektor PA-MV und den einzelnen Protokoll-Merkmalsvektoren PR-MV ermittelt. Derartige Ähnlichkeits-Algorithmen SA sind aus dem Stand der Technik bekannt. Beispielsweise kann der Ähnlichkeits-Algorithmus SA einen Kosinus-Ähnlichkeits-Algorithmus oder Euclidean distance (L2 Norm) oder Dot Product Similarity usw. umfassen.

Mittels des Ähnlichkeits-Algorithmus SA wird eine Empfehlungsinformation EI generiert und/oder ermittelt. Die Empfehlungsinformation EI umfasst zumindest ein Magnetresonanz-Protokoll MP, dessen Protokoll-Merkmalsvektor PR-MV eine Maximal-Übereinstimmung mit dem Patienten-Merkmalsvektor PA-MV aufweist. Mittels des Ähnlichkeits-Algorithmus SA wird für alle Protokoll-Merkmalsvektoren PR-MV eine Ähnlichkeit mit dem Patienten-Merkmalsvektor PA-MV ermittelt. Derjenige Protokoll-Merkmalsvektor PR-MV eines Magnetresonanz-Protokolls MP mit der größten Übereinstimmung mit dem Patienten-Merkmalsvektor PA-MV weist eine Maximal-Übereinstimmung auf.

Bevorzugt weist die Empfehlungsinformation EI mehrere Magnetresonanz-Protokolle MP auf, wobei die mehreren Magnetresonanz-Protokolle MP hinsichtlich einer Übereinstimmung zwischen dem Patienten-Merkmalsvektor PA-MV und dem Protokoll-Merkmalsvektor PR-MV des jeweiligen Magnetresonanz-Protokolls MP sortiert sind. Insbesondere sind die mehreren Magnetresonanz-Protokolle MP derart sortiert, dass die Magnetresonanz-Protokolle MP mit der größten Übereinstimmung mit dem Patienten-Merkmalsvektor PA-MV zuerst aufgeführt sind.

In einem weiteren, sechsten Verfahrensschritt 105 erfolgt ein Bereitstellen der Empfehlungsinformation EI. Die Bereitstellung der Empfehlungsinformation EI erfolgt bevorzugt mittels einer Ausgangsschnittstelle 12 der Recheneinheit 10.

Nach dem Bereitstellen der Empfehlungsinformation EI kann es in einem weiteren, daran anschließenden siebten Verfahrensschritt 106 vorgesehen sein, dass die bereitgestellte Empfehlungsinformation EI an einen Benutzer ausgegeben wird. Die Ausgabe erfolgt bevorzugt mittels einer visuellen Ausgabeeinheit 13, beispielsweise einem Monitor und/oder einem Display. Die Ausgabeeinheit 13 ist bevorzugt von der Recheneinheit 10, insbesondere einer Benutzerschnittstelle 14 der Recheneinheit 10, umfasst. Dabei kann die Ausgabe lediglich die Empfehlungsinformation EI, beispielsweise das eine empfohlene Magnetresonanz-Protokoll MP oder einer Liste mit den empfohlenen Magnetresonanz-Protokollen MP, umfassen. Zudem kann die Ausgabe der Empfehlungsinformation EI auch eine Voreinstellung einer Protokollauswahl bei einer Planung der Magnetresonanzuntersuchung umfassen. Bei einer Übernahme der Empfehlung kann das medizinische Bedienpersonal die Vorauswahl einfach bestätigen und das empfohlene Magnetresonanz-Protokoll MP ist ausgewählt. Alternativ hierzu kann der Benutzer, insbesondere das medizinische Bedienpersonal, die Vorauswahl auch ablehnen, sofern er mit der Protokollauswahl nicht einverstanden ist.

Die Empfehlungsinformation EI und/oder ein Feedback des medizinischen Bedienpersonals zur Empfehlungsinformation EI kann in den Verfahrensschritten 105 und 106 auch der Recheneinheit 10, insbesondere dem LLM und/oder dem Ähnlichkeits-Algorithmus SA, wieder zur Verfügung gestellt werden, um eine Ermittlung des Patienten-Merkmalsvektors PA-MV und/oder der Protokoll-Merkmalsvektoren PR-MV und/oder der Empfehlungsinformation EI zu verbessern.

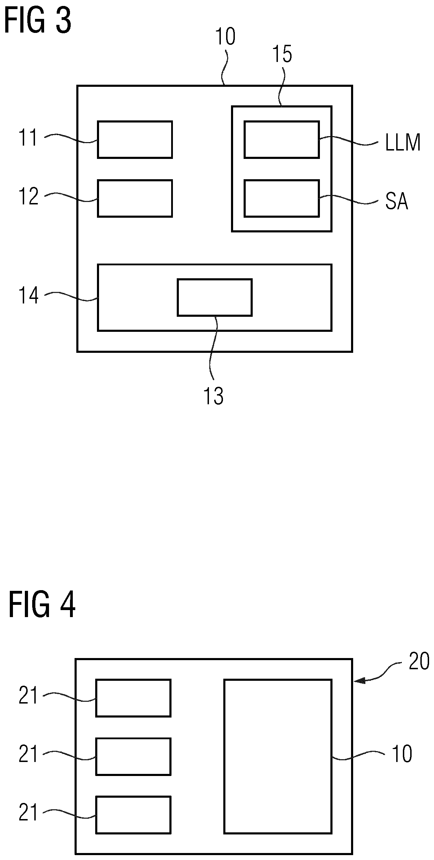

In Fig. 3 ist die Recheneinheit 10 näher dargestellt. Die Recheneinheit 10 ist zum Steuern des Verfahrens zu einem Bereitstellen einer Empfehlungsinformation EI für zumindest ein Magnetresonanz-Protokoll MP für eine Magnetresonanzuntersuchung an einem Patienten ausgebildet. Hierzu weist die Recheneinheit 10 eine Eingangsschnittstelle 11, ein Analysemodul 15 und eine Ausgangsschnittstelle 12 auf.

Die Eingangsschnittstelle 11 ist dazu ausgebildet, auf zumindest eine Datenbank 21, insbesondere eine digitale Datenbank 21, oder auch mehrere Datenbanken 21, bevorzugt digitale Datenbanken 21, zuzugreifen. In diesen Datenbanken 21 sind die untersuchungsrelevanten Patienteninformationen URP und/oder die untersuchungsrelevanten Anwenderinformationen URA und die Protokollinformationen PI zu den einzelnen Magnetresonanz-Protokollen MP gespeichert. Beispielsweise umfasst eine erste Datenbank 21 eine Patientendatenbank und/oder einer RIS-Datenbank, in der die untersuchungsrelevanten Patienteninformationen URP gespeichert sind. Bevorzugt sind in der ersten Datenbank 21 alle untersuchungsrelevanten Patienteninformationen URP gespeichert. Beispielsweise können in einer zweiten Datenbank 21 die untersuchungsrelevanten Anwenderinformationen URA gespeichert sein. In einer weiteren, dritten Datenbank 21 können beispielsweise die Protokollinformationen Pl zu den unterschiedlichen Magnetresonanz-Protokollen MP gespeichert sein. Mittels der Eingangsschnittstelle 11 können die untersuchungsrelevanten Patienteninformationen URP und/oder die untersuchungsrelevanten Anwenderinformationen URA und/oder die Protokollinformationen PI zu den unterschiedlichen Magnetresonanz-Protokollen MP empfangen werden.

Das Analysemodul 15 umfasst bevorzugt das trainierte LLM und den Ähnlichkeits-Algorithmus SA. Die Recheneinheit 10 kann dabei einen Prozessor aufweisen, der von dem Analysemodul 15 umfasst ist. Das Analysemodul 15 ist dazu ausgebildet, mittels des trainierten LLMs einen Patienten-Merkmalsvektor PA-MV anhand der untersuchungsrelevanten Patienteninformation URP und/oder der zumindest einer untersuchungsrelevanten Anwenderinformation URA bereitzustellen und für die Protokollinformationen PI eines jeden Magnetresonanz-Protokolls MP einen Protokoll-Merkmalsvektor PR-MV bereitzustellen. Zudem ist das Analysemodul 15 weiterhin dazu ausgebildet, mittels des Ähnlichkeits-Algorithmus SA eine Empfehlungsinformation EI anhand des Patienten-Merkmalsvektors PA-MV und der Protokoll-Merkmalsvektoren PR-MV zu ermitteln.

Die Ausgangsschnittstelle 12 der Recheneinheit 10 ist zu einem Bereitstellen der Empfehlungsinformation EI ausgebildet.

Bevorzugt weist die Recheneinheit 10 zu einem Steuern und/oder Ausführen des Verfahrens zu einem Bereitstellen einer Empfehlungsinformation EI für zumindest ein Magnetresonanz-Protokoll MP für eine Magnetresonanzuntersuchung ein Computerprogrammprodukt mit Programmelementen auf. Die Programmelemente veranlassen die Recheneinheit 10, die Schritte des Verfahrens zu einem Bereitstellen einer Empfehlungsinformation EI für zumindest ein Magnetresonanz-Protokoll MP für eine Magnetresonanzuntersuchung auszuführen, wenn die Programmelemente in einen Speicher der Recheneinheit 10 geladen werden.

In Fig. 4 ist ein System 20 zu einem Bereitstellen einer Empfehlung zumindest eines Magnetresonanz-Protokolls MP für eine Magnetresonanzuntersuchung an einem Patienten dargestellt. Das System 20 umfasst die Recheneinheit 10, die bereits in der Beschreibung zu Fig. 3 näher beschrieben wurde. Des Weiteren weist das System 20 zumindest eine digitale Datenbank 21 auf. Im vorliegenden Ausführungsbeispiel weist das System 20 drei digitale Datenbanken 21 auf. In einer ersten digitalen Datenbank 21, beispielsweise in einer Patientendatenbank und/oder einer RIS-Datenbank, sind die untersuchungsrelevanten Patienteninformationen URP gespeichert. In einer zweiten digitalen Datenbank 21 sind die untersuchungsrelevanten Anwenderinformationen URA gespeichert. In einer dritten digitalen Datenbank 21 sind die Protokollinformationen PI zu den unterschiedlichen Magnetresonanz-Protokollen MP gespeichert. Das System 20 kann weiterhin eine Ausgabeeinheit zu einer Ausgabe der Empfehlungsinformation EI aufweisen.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Computerimplementiertes Verfahren zu einem Bereitstellen einer Empfehlungsinformation für zumindest ein Magnetresonanz-Protokoll für eine Magnetresonanzuntersuchung an einem Patienten, umfassend die folgenden Verfahrensschritte:
- Bereitstellen von zumindest einer untersuchungsrelevanten Patienteninformation und/oder zumindest einer untersuchungsrelevanten Anwenderinformation,
- Bereitstellen von Protokollinformationen zu unterschiedlichen Magnetresonanz-Protokollen aus einer Gesamtheit an zur Verfügung stehenden Magnetresonanz-Protokollen,
- Anwenden eines trainierten Sprachen-Modells auf die zumindest eine untersuchungsrelevanten Patienteninformation und/oder die zumindest eine untersuchungsrelevanten Anwenderinformation, wobei mittels des trainierten Sprachen-Modells ein Patienten-Merkmalsvektor ermittelt und bereitgestellt wird,
- Anwenden des trainierten Sprachen-Modells auf Protokollinformationen der unterschiedlichen Magnetresonanz-Protokolle, wobei mittels des trainierten Sprachen-Modells ein Protokoll-Merkmalsvektor für jedes Magnetresonanz-Protokoll der unterschiedlichen Magnetresonanz-Protokolle ermittelt und bereitgestellt wird,
- Anwenden eines Ähnlichkeits-Algorithmus auf den Patienten-Merkmalsvektor und den unterschiedlichen Protokoll-Merkmalsvektoren und Ermitteln einer Empfehlungsinformation umfassend zumindest ein Magnetresonanz-Protokoll, wobei der Protokoll-Merkmalsvektor des zumindest einen Magnetresonanz-Protokolls der Empfehlungsinformation eine Maximal-Übereinstimmung zu dem Patienten-Merkmalsvektor aufweist, und,
- Bereitstellen der Empfehlungsinformation.

2. Verfahren nach Anspruch 1,
**dadurch gekennzeichnet, dass** die zumindest eine untersuchungsrelevante Patienteninformation zumindest eine der folgenden Informationen umfasst:
- Daten und/oder Informationen einer vorhergehenden Untersuchung des Patienten und/oder eines bisherigen Krankheitsverlaufs des Patienten,
- Daten und/oder Informationen, die von einem medizinischen Personal in Vorbereitung zu der anstehenden Magnetresonanzuntersuchung des Patienten erstellt wurden, und/oder
- Notizen und/oder Bemerkungen, die von einem medizinischen Personal zu dem Patienten erstellt wurden.

3. Verfahren nach einem der vorhergehenden Ansprüche,
**dadurch gekennzeichnet, dass** die zumindest eine untersuchungsrelevante Anwenderinformation eine betreiberabhängige und/oder lokale Anweisung und/oder Vorschrift zur Magnetresonanzuntersuchung umfasst.

4. Verfahren nach einem der vorhergehenden Ansprüche,
**dadurch gekennzeichnet, dass** die zumindest eine untersuchungsrelevante Patienteninformation und/oder die zumindest eine untersuchungsrelevante Anwenderinformation einen Freitext umfasst.

5. Verfahren nach einem der vorhergehenden Ansprüche,
**dadurch gekennzeichnet, dass** das Bereitstellen der zumindest einen untersuchungsrelevanten Patienteninformation und/oder der zumindest einen untersuchungsrelevanten Anwenderinformation ein Generieren einer Textzeichenfolge aus der zumindest einen untersuchungsrelevanten Patienteninformation und/oder der zumindest einen untersuchungsrelevanten Anwenderinformation umfasst.

6. Verfahren nach einem der vorhergehenden Ansprüche,
**dadurch gekennzeichnet, dass** das Bereitstellen von Protokollinformationen zu unterschiedlichen Magnetresonanz-Protokollen aus der Gesamtheit an zur Verfügung stehenden Magnetresonanz-Protokollen eine Beschränkung von Protokollinformationen zu unterschiedlichen Magnetresonanz-Protokollen einer Protokoll-Gruppe umfasst.

7. Verfahren nach einem der vorhergehenden Ansprüche,
**dadurch gekennzeichnet, dass** das Bereitstellen von Protokollinformationen zu unterschiedlichen Magnetresonanz-Protokollen ein Generieren einer Textzeichenfolge aus jeder Protokollinformation eines Magnetresonanz-Protokolls umfasst.

8. Verfahren nach einem der Ansprüche 5 und/oder 7,
**dadurch gekennzeichnet, dass** das trainierte Sprachen-Modell ein auf einem neuronalen Netzwerk basierendes Encoder-Netzwerk umfasst, das zu einer Kodierung der Textzeichenfolgen ausgebildet ist.

9. Verfahren nach einem der vorhergehenden Ansprüche,
**dadurch gekennzeichnet, dass** die Empfehlungsinformation mehrere Magnetresonanz-Protokolle umfasst, wobei die mehreren Magnetresonanz-Protokolle hinsichtlich einer Übereinstimmung zwischen dem Patienten-Merkmalsvektor und dem Protokoll-Merkmalsvektor des jeweiligen Magnetresonanz-Protokolls sortiert sind.

10. Verfahren nach einem der vorhergehenden Ansprüche,
**dadurch gekennzeichnet, dass** in einem weiteren Verfahrensschritt eine Ausgabe der bereitgestellten Empfehlungsinformation an einen Benutzer erfolgt.

11. Verfahren nach Anspruch 10,
**dadurch gekennzeichnet, dass** die Ausgabe der Empfehlungsinformation eine Voreinstellung einer Protokollauswahl bei einer Planung der Magnetresonanzuntersuchung des Patienten umfasst.

12. Verfahren nach einem der vorhergehenden Ansprüche,
**dadurch gekennzeichnet, dass** das Sprachen-Modell anhand der ermittelten Empfehlungsinformation nachtrainiert wird.

13. Recheneinheit zu einem Regeln und/oder Steuern eines Verfahrens nach einem der vorhergehenden Ansprüche, umfassend:
- eine Eingangsschnittstelle, die dazu ausgebildet ist, auf zumindest eine Datenbank zuzugreifen, in der die zumindest eine untersuchungsrelevante Patienteninformation und/oder zumindest eine untersuchungsrelevanten Anwenderinformation und/oder die Protokollinformationen zu unterschiedlichen Magnetresonanz-Protokollen gespeichert sind, um die zumindest eine untersuchungsrelevante Patienteninformation und/oder die zumindest eine untersuchungsrelevanten Anwenderinformation und/oder die Protokollinformationen zu unterschiedlichen Magnetresonanz-Protokollen zu empfangen,
- ein Analysemodul, das dazu ausgebildet ist, mittels eines trainierten Sprachen-Modells einen Patienten-Merkmalsvektor anhand der zumindest einen untersuchungsrelevanten Patienteninformation und/oder der zumindest einen untersuchungsrelevanten Anwenderinformation bereitzustellen und mittels des trainierten Sprachen-Modells für die Protokollinformationen eines jeden Magnetresonanz-Protokolls einen Protokoll-Merkmalsvektor bereitzustellen, wobei das Analysemodul weiterhin dazu ausgebildet ist, mittels eines Ähnlichkeits-Algorithmus eine Empfehlungsinformation anhand des Patienten-Merkmalsvektors und der Protokoll-Merkmalsvektoren zu ermitteln, und
- eine Ausgangsschnittstelle, die zu einem Bereitstellen der Empfehlungsinformation ausgebildet ist.

14. System zu einem Bereitstellen einer Empfehlung zumindest eines Magnetresonanz-Protokolls für eine Magnetresonanzuntersuchung an einem Patienten, umfassend:
- eine Recheneinheit nach Anspruch 13, und
- zumindest eine digitale Datenbank, in der die zumindest eine untersuchungsrelevanten Patienteninformation und/oder die zumindest eine untersuchungsrelevanten Anwenderinformation und/oder die Protokollinformationen gespeichert sind.

15. Computerprogrammprodukt mit Programmelementen, die eine Recheneinheit veranlassen, die Schritte des Verfahrens zu einem Bereitstellen einer Empfehlungsinformation für zumindest ein Magnetresonanz-Protokoll für eine Magnetresonanzuntersuchung an einem Patienten nach Anspruch 1 bis 12 gemäß dem Verfahrensaspekt auszuführen, wenn die Programmelemente in einen Speicher der Recheneinheit geladen werden.
